Europäisches Patentamt

**European Patent Office**

Office européen des brevets

(19)

(11) Publication number: **0 160 390**
**A2**

(12) # EUROPEAN PATENT APPLICATION

(21) Application number: **85302096.4**

(22) Date of filing: **26.03.85**

(51) Int. Cl.⁴: **A 01 H 5/10**
**A 01 G 7/00**

(30) Priority: **16.04.84 US 600855**

(43) Date of publication of application:
**06.11.85 Bulletin 85/45**

(84) Designated Contracting States:
**AT DE FR IT**

(71) Applicant: **STAUFFER CHEMICAL COMPANY**

**Westport Connecticut 06881(US)**

(72) Inventor: **Lowe, Keith Sands**
**3000 Otis Drive**
**Alameda California 94501(US)**

(74) Representative: **Smith, Sydney et al,**
**Elkington and Fife High Holborn House 52/54 High**
**Holborn**
**London WC1V 6SH(GB)**

(54) **Embryogenic callus and cell suspension of inbred corn.**

(57) Embryogenic callus of corn inbred B73, embryogenic cell suspensions of corn inbred B73, clones of the embryogenic callus and cell suspensions of corn inbred B73, corn plants and the seed thereof regenerated from the embryogenic callus, embryogenic cell suspensions and clones of the embryogenic callus and embryogenic cell suspensions, and progeny of the regenerated corn plants including mutant and variant progeny are claimed.

EP 0 160 390 A2

## EMBRYOGENIC CALLUS AND CELL SUSPENSION OF INBRED CORN

### Field of the Invention

The invention relates to embryogenic callus of corn inbred B73 – a major commercial inbred corn variety. The invention also relates to cell suspensions derived from embryogenic callus of corn inbred B73. The cell suspensions may be characterized as uniform mixtures of embryogenic and other cells. Unexpectedly, the cell suspensions according to the invention give rise to embryos of corn inbred B73 under certain culture conditions described hereinbelow. The invention furthermore relates to viable seed-producing plants of corn inbred B73 regenerated from the embryogenic callus or embryogenic cell suspensions according to the invention. Lastly, the invention concerns mutant and/or recombinant progeny of embryogenic callus or embryogenic cell suspension and corn plants regenerated therefrom. Specific examples of the embryogenic callus of corn inbred B73, from which embryogenic cell suspensions may be derived, have been deposited in the American Type Culture Collection under ATCC accession number 40116 on April 14, 1984.

### Relevant Art

Zea mays L. or corn is a major worldwide cereal crop. In the continental United States alone, an estimated 82 million acres of corn is planted yearly. A survey of the U. S. corn germ plasm base conducted in 1979 accounting for 1.3 billion pounds of seed corn, determined that corn inbred B73 was used in the production of approximately 178.5 million pounds of corn hybrid seed. This amount reflects approximately 14% of the total corn seed required for the 1980 planting of 82 million acres of corn in the United States. B73 was used to produce more hybrid corn seed than any other corn inbred line in the 1979 survey. As the major inbred corn line used in hybrid corn seed production, B73 is a commercially important corn inbred. Successful efforts to improve B73 are likely to have a major impact on the commercial corn seed business.

Current methods for improving inbred corn lines are time consuming, labor intensive, and risky. Thus, methods that reduce any of

0160390

these problems would represent an advance in the cereal breeding arts. One method for inducing changes in an inbred corn seed line is accomplished by exposing inbred seed corn to a mutagen, which may be ionizing radiation or a mutagenic chemical, growing the seed out, and screening the corn plants for a desired characteristic such as decreased height or pathogen resistance. Seed from plants displaying the desired characteristics are then replanted, rescreened and the seed from plants passing the screen are retained. After several years and many grow-outs of the seed, a sufficient seed stock of the inbred displaying the desired characteristic is accumulated for commercial breeding.

Several problems attend the method of obtaining a modified inbred corn seed line as described above. Inbred corn lines are highly homozygous, or pure-bred. Such inbred lines frequently display low vigor, and as a result frequently produce few seed. If seed production is too low, successful large-scale production of seed derived from a single plant of a modified inbred line may not be possible at all. Even if the modified inbred is vigorous and produces adequate numbers of fertile seed, several years and many acres of land are required to produce enough seeds to make commercial breeding possible.

Other approaches to the propagation of desirable modified inbreds have also been suggested and rely upon plant tissue culture techniques. In some of these approaches, cell cultures are subjected to mutagenic ionizing radiation. The desirable characteristic is selected or screened in cell culture. Cells having the desirable characteristic are propagated in culture and ultimately regenerated to form seed-bearing plants (see e.g., Bottino P. J., "The Potential of Genetic Manipulation of Plant Cell Cultures for Plant Breeding," Radiation Botany, 15:1-16 (1975).

The feasibility of using selection techniques on callus cultures of certain non-commercial corn inbreds and the regeneration of plants producing seed that carry the characterstic selected in tissue culture has been demonstrated by Hibbert et al. (see, "Selection and Characterization of a Feedback - Insensitive Tissue Culture of Maize," Planta, 148:183-187 (1980).)

Prior to the development of the embryogenic callus line of corn inbred B73 of the instant invention, no friable embryogenic callus culture or embryogenic cell suspension culture of a commercially significant inbred corn line has been described. With respect to embryogenic callus lines of noncommercial corn inbreds, C.E. Green has described a friable callus of corn inbred A188 capable of regenerating plants by somatic embryogenesis (see, Green, C.E., "Somatic Embryogenesis and Plant Regeneration From the Firable Calus of Zea mays L." in Proc. 5th Int'l. Cong. Plant Tissues and Cells, Akio Fujiwara ed., Japanese Association of Plant Tissue Culture, pub. Tokyo, Japan, 1982). Embyrogenic, friable A188 callus according to Green can be derived from spontaneous sectors of organogenic callus. Organogenic callus according to Green can be obtained as follows. Immature zygotic embryos from the seed of sibling pollinated A188 are removed from the seed and placed in a Murashige and Skoog (MS, see Murashige and Skoog, Physiologia Plantarium, 15:426 (1962)) medium, with 0.5 mg/l 2,4-D, 1 mM L-asparagine, and 0.7% bactoagar. The immature zygotic embryos are incubated at 28°C with 16 hours light per day. (See Springer, W.D. et al., 1979, "A Histological Examination of Tissue Culture Initiation From Immature Embryos of Maize", Protoplasma, 101, 269-281.) The scutellum of the primary zygotic embryo proliferates rapidly and forms an organogenic callus culture. The epithelium of the scutellum becomes highly meristematic. Apical meristems form at the surface of the culture which is characterized by an extended cambial zone. Because no root primordia are associated with the shoot primordia, the callus is considered organogenic.

Green's embryogenic callus arises spontaneously as sectors growing from the established organogenic callus or can be induced from primary cultures with proline. Proline, however, does not appear to induce embryogenic callus in B73. Green's embryogenic callus appears undifferentiated, highly friable, has little organization on visual inspection and is fast growing, requiring sub-culturing at least every two weeks. The embryogenic callus culture described by Green develops to the coleoptilar stage on MS or N6 medium with 2% sucrose and 0.5 to 1.0 mg/l 2,4-D. Normal development to the mature embryo stage is possible if the embryos are transferred to N6 medium with 6% sucrose and no 2,4-D. The mature embryos can then be induced to germinate on MS or N6 medium containing 2% sucrose without hormones.

Green also describes suspension cultures of the embryogenic line of A188 and discloses that such suspension cultures can be maintained in MS medium containing 1 mg/l 2,4-D with 2% sucrose. When aliquots of the suspension cultures are plated onto solidified MS medium containing 2% sucrose, a callus forms from the plated suspension and embryos form on the callus surface. These embryos are transferred to MS or N6 medium with 6% sucrose for further development. After 2 weeks on medium with 6% sucrose these are transferred to low sucrose, hormone-free medium for embryo generation. A188 plants may be routinely regenerated from these embryos.

A non-regenerating cell line of corn inbred B73 is known (see Potrykus et al, Theoretical Applied Genetics, 54:209, 1979). This cell line in suspension culture was established from a primary culture of protoplasts from plant tissues of the B73 inbred. The cells are characterized as growing well in suspension culture and easily maintainable on agar medium. Furthermore, the cells can be enzymatically turned into protoplasts, which in turn regenerate walls and become proliferating cells again. Significantly, the cells of this line have never been induced to regenerate plants and moreover display ploidy abnormalities, a characteristic well known in other non-regenerating diploid plant cell lines. (See M. G. Meadows, "Characterization of Cells and Protoplasts of the B73 Maize Cell Line" Plant Science Letters, 28:337-348(1982/83). Other attempts to achieve plant regeneration from established B73 callus have been similarly unsuccessful. (See Bartkowiak, E., "Tissue Culture of Maize Plantlet Regeneration From Scutella Callus," Genetica Polonica, Vol. 23:93-101 (1982)).

The ability to culture somatic cells of inbred plants in vitro enables the plant breeder to apply the techniques of microbial genetics to specific breeding problems in crop plants. Included in the genetical approaches that have application to plant breeding are: selection of mutations in cell cultures, studying host-pathogen interrelationships, transfer of genetic information into cells through uptake of exogenous heterologus (see Szoka et al., U. S. Patent 4,394,448) or homologous (see IPRI European Patent Application No. 90033A) DNA. Furthermore, somatic cell culture enables the plant breeder to develop, select and propagate somaclonal variants having novel or useful agronomic characteristics.

The major obstacle to the application of these techniques to commercial corn inbred B73 and some other commercial inbreds has, until now, been the inability to provide a tissue culture or cell suspension that can regenerate whole plants. The embryogenic corn callus line and corn cell line derived therefrom provided by the instant invention makes it possible to apply somatic cell genetic techniques to a major commercial corn line for the first time. The particular uses to which a cereal, and particularly a corn cell line such as the one described herein, can be put are outlined in numerous articles (see e.g., Bottino, P. J. "Potential of Genetic Manipulation in Plant Cell Culture," Radiation Botany, Vol 15: 1-16 (1975) and Vasil, I.E., "Plant Cell Culture and Somatic Cell Genetics of Cereals and Grasses," Plant Improvement and Somatic Cell Genetics, Vasil, Ed., Academic Press, N.Y., 1st Ed., (1983) and "Tissue Culture in the Production of Novel Disease-Resistant Crop Plants," Biol. Rev., 54:329-345 (1979)).

These uses include selection at the cellular level of mutations for resistance to toxic substances, such as herbicides and substances produced by plant pathogens. Since the selections are carried out at the cellular level, it is likely that whole plants regenerated from the cells will show the selected characteristic. It is significant that such a system would allow plant breeders to select (or screen) for the desired characteristic from among thousands of cells in a single culture dish or flask, whereas a large field plot would be required to select or screen a corresponding number of seed grown plants according to traditional plant breeding methods.

It has, for example, been demonstrated that corn plants derived from Black Mexican Sweet corn, a non-commercial cultivar, can be regenerated from tissue cultures that have been selected for resistance to lysine and threonine and that tissues of the regenerated plant likewise produce corn plant tissue that is resistant to lysine and threonine. See Hibbert, K.A et al., "Selection and Characterization of a Feedback - Insensitive Tissue Culture of Maize," Planta, 148:183-187 (1980).

The utility of such selections carried out in callus cultures has also been demonstrated by the regeneration from callus culture of corn plants resistant to southern corn leaf blight, a plant disease caused by a pathotoxin produced by the plant pathogen Helmenthosporium maydis race T. By exposing corn callus to the pathotoxin produced by the plant pathogen, resistant callus was selected. Significantly, the progeny of certain resistant plants were also resistant to the toxin and plant pathogen. Thus callus culture of a corn cell line is demonstrably effective in the development of pathogen resistant corn seed. (See Gengenbach et al., "Inheritance of Selected Pathotoxin Resistance in Maize Plants Regenerated From Cell Cultures," Proc. Nat'l. Acad. Sci. USA, 74:5113-5117 (1977)).

## Brief Description of the Drawings

Figure 1 is a representation of an isozyme pattern for esterase obtained by starch gel electrophoresis as further described in Example 7.

Figure 2 is a representation of an isozyme pattern for alcohol dehydrogenase obtained by starch gel electrophoresis as further described in Example 7.

Figure 3 is a representation of an isozyme pattern for glutamate dehydrogenase obtained by starch gel electrophoresis as further described in Example 7.

Figure 4 is a representation of an isozyme pattern for $\beta$-glucosidase obtained by starch gel electrophoresis as further described in Example 7.

## Summary of the Invention

The present invention comprises tissue cultures of corn inbred line B73. These tissue cultures are characterized as embryogenic callus cultures of corn inbred B73 and cell suspension cultures derived from the embryogenic callus culture of corn inbred B73. As used herein, the term "embryogenic callus" is defined as a friable callus having no visible organization on maintenance medium but on regeneration medium organizes into discrete globular structures, a portion of which germinate to form plants.

As used herein the term "variation" is defined as phenotypic changes that are: stable, i.e., they persist in the absence of the event that induced the change; and heritable, i.e., the new phenotype is transmitted to daughter cells when they divide. Changes in phenotype that persist only so long as the cells or tissues are maintained in a new environment are referred to as physiological responses.

"Genetic variation" is used herein to describe heritable variation that is sexually transmitted to progeny of plants regenerated from cultured cells or tissues. The term "mutant" is reserved for the special case of genetic variation in which a trait is transmitted meiotically according to well-established laws of inheritance. When the nature of the heritable change is not known, the term "variant" is used.

As used herein the term "regenerated", referring to plants, means plants derived from cell and tissue culture. The term "progeny" means plants obtained by self-fertilization, sibling-fertilization, backcrossing or out-crossing of the regenerated plants.

As used herein the term "clone" refers to callus or cell suspensions propagated from an established cell suspension or callus line.

The embryogenic callus culture of corn inbred B73 characteristically is a collection of substantially isodiametric cells which grow in clumps or aggregates of cells on solid maintenance medium such as MS medium or N6 medium that has been supplemented with 0.25 to 1.0 mg/l 2,4-D and 2% (wt/vol) sucrose. On maintenance medium the embryogenic callus is friable, i.e., easily broken into smaller clumps of cells and single cells. Under the light microscope, no continuous epidermal layer is visible in the embryogenic callus. In general, the embryogenic callus is yellow-brown in color. Growth of the embryogenic callus is rapid, as measured in fresh weight accumulation; the embryogenic callus grows about 3 times faster than the organogenic callus culture from which it arose. As a consequence of its growth rate, the embryogenic callus needs to be transferred to new medium every 1-2 weeks.

The embryogenic callus is further characterized by the absence of differentiated plant structure such as small shoots or their primordia, roots or their primordia, and meristems. A characteristic of cell suspensions derived from the callus is the production of a mucilagenous polysaccharide in media containing sucrose and 2,4-D. This polysaccharide has been identified as a glactose-mannose polymer.

The most notable characteristic of the embryogenic callus is its formation of embryo-like structures. The embryo-like structures are compact and globular. By transferring the globular structures to a hormone-free medium such as MS or N6 medium, some of these globular structures can be induced to form plants. The plants can be transferred to soil and fertile B73 corn plants are obtained. The progeny of these regenerated plants are substantially identical in form to seed-grown B73 plants. Thus, the invention encompasses corn plants regenerated from an embryogenic callus of corn inbred B73 and the progeny of the regenerated corn plants.

The invention further comprises embryogenic cell suspension cultures of embryogenic callus culture of corn inbred B73. Unexpectedly, these cell cultures retain the ability to regenerate viable B73 corn plants. B73 cell suspensions capable of regenerating whole plants are initiated from friable B73 callus maintained on MS medium. The friable callus is transferred into a medium suitable for maintaining suspension culture, e.g. MS medium with 2 mg/l 2,4-D, and is placed on a gyrotary shaker at 130 rpm at 28°C. Cell suspensions are induced to form globular embryos by plating on solidified MS or N6 medium containing no hormones and 2-6% sucrose. Globular embryos are transferred to solidified MS or N6 medium containing no hormones, 2% sucrose and optionally charcoal added to adsorb residual hormones, if any, and plantlets form on this medium. Thus the invention encompasses corn plants regenerated from an embyrogenic cell suspension of corn inbred B73 and the progeny of the regenerated corn plants.

The invention will be better understood from the following examples which are intended by the inventor to be exemplary only and nonlimiting.

## EXAMPLE 1

### Initiation of Organogenic Culture

Plants from maize inbred line B73 were grown to maturity in a greenhouse supplemented with metal halide lights (15 hours/day) and maintained at 30°C during the day and 21°C at night. These plants were self- or sibling-pollinated. Immature zygotic embryos 1.0-2.0 millimeters (mm) in length (12-14 days post pollination) were aseptically removed from surface sterilized kernels and placed flat side down on the initiation medium which contained MS salts and MS vitamins, 150 mg/1 asparagine, 0.6% agar, 0.5 mg/1 2,4-D and 12% sucrose. All culture medium had been adjusted to a pH of 5.8 prior to autoclaving. Cultures were incubated in the dark at 28°C for 2-3 weeks, when a compact white tissue proliferated from the coleorhizal end of the scutellum. The zygotic embryonic shoot axis was not part of the proliferating tissues. The white compact tissue which was composed of small parenchyma-like cells with meristematic regions was covered by an epidermal layer. Numerous root meristems, occasional shoots and starch grains were seen in this tissue. White compact tissue was removed from the embryos, transferred to a maintenance medium, which was the same as the initiation medium but included 0.5-1.0 mg/1 2,4-D and 2% sucrose, and cultured under Agrolites (1500 lux) at 28°C. Cultures were transferred to fresh medium every 10-20 days.

The compact tissue produced on the initiation medium became extremely heterogeneous when transferred to the maintenance medium. Numerous shoots, roots, as well as friable and compact green tissues became visible. Leafy and rooty tissues were carefully removed and discarded from the compact organogenic tissue at each transfer.

After one year of continuous selection against non-regenerating regions and roots, the resulting organogenic tissue was dark green, compact, convoluted and covered by a well-defined epidermis. This epidermal layer remained continuous even in zones that produced shoot meristems. Meristematic centers were common beneath the epidermal layer. This tissue was characterized by an extremely slow growth rate which could be increased by the addition of 9 mg/l zeatin. The addition of zeatin also reduced the quantity of tissues normally selected against.

## EXAMPLE 2

### Initiation and Selection of Embryogenic Callus

A friable, fast-growing callus was selected from the organogenic tissue after over one year in culture in the following manner. The organogenic callus from which the embryogenic callus was isolated was transferred to maintenance medium contining MS salts and MS vitamins, 150 mg/l asparagine, 1 mg/l 2,4-D, 1 mg/l 3,5-dichlorophenxy acetic acid (3,5-D), 2% sucrose and 0.6% washed agar. The organogenic callus was transferred after 3 weeks to fresh medium having the same components.

After a total of 5 weeks on the medium, the embryogenic callus appeared as small isodiametric cells having dense cytoplasm and large nuclei on microscopic examination. The sectors were embedded in a mucilagenous matrix. The sectors and mucilagenous matrix were removed from the surrounding compact organogenic callus with sterile tweezers or were removed by scraping with a sterile scapel. The sectors and mucilagenous matrix were placed on maintenance medium as described above but lacking 3,5-D. Friable embryogenic callus grew approximately 3 times faster than the organogenic tissue on the maintenance medium with 3,5-D. Although the embryogenic callus was initially isolated from an organogenic callus that had been grown on maintanence medium including 3,5-D, embryogenic sectors have been subsequently isolated from organogenic callus that has been maintained without 3,5-D. These isolates, however, tend to rapidly organize and cannot be maintained.

## EXAMPLE 3

### Initiation of Suspension Cultures

One and seventy-seven one-hundredths gram (1.77 g) of friable callus tissue from corn inbred B73 was inoculated into 25 ml of MS medium having the following components:

magnesium sulfate·seven hydrate ($MgSO_4 \cdot 7H_2O$),
calcium chloride·dihydrate ($CaCl_2 \cdot 2H_2O$),
potassium nitrate ($KNO_3$),

ammonium nitrate ($NH_4NO_3$),
potassium phosphate ($KH_2PO_4$),
manganese sulfate·four hydrate ($MnSO_4 \cdot 4H_2O$),
zinc sulfate·seven hydrate ($ZnSO_4 \cdot 7H_2O$),
cupric sulfate·five hydrate ($CuSO_4 \cdot 5 H_2O$),
cobalt chloride·six hydrate ($CoCl_2 \cdot 6H_2O$
potassium iodide (KI),
boric acid ($H_3BO_3$),
sodium molybdinum oxide·dihydrate ($Na_2MoO_4 \cdot 2H_2O$),
ferrous sulfate·seven hydrate ($FeSO_4 \cdot 7H_2O$), and
sodium ethylenediaminotetracetic acid ($Na_2EDTA$).

In general, as used in the invention, the exact concentration of the salts can be varied within limits without departing from the invention. To standardize the making of the media, however, the concentrations of the above listed minimal salts are as follows:

| | |
|---|---|
| $MgSO_4 \cdot 7H_2O$ | 370 milligrams/liter (mg/1) |
| $CaCl_2 \cdot 2H_2O$ | 440 mg/1 |
| $KNO_3$ | 1900 mg/1 |
| $NH_4NO_3$ | 1650 mg/1 |
| $KH_2PO_4$ | 170 mg/1 |
| $MnSO_4 \cdot 4H_2O$ | 22.3 mg/1 |
| $ZnSO_4 \cdot 7H_2O$ | 8.6 mg/1 |
| $CoSO_4 \cdot 5H_2O$ | 0.025 mg/1 |
| $CoCl_2 \cdot 6H_2O$ | 0.025 mg/1 |
| KI | 0.83 mg/1 |
| $H_3BO_3$ | 6.2 mg/1 |
| $Na_2MoO_4 \cdot 2H_2O$ | 0.25 mg/1 |
| $FeSO_4 \cdot 7H_2O$ | 28.75 mg/1 |
| $Na_2EDTA$ | 37.25 mg/1 |

The medium further contains the following vitamins, hormones and carbohydrate sources:

| | |
|---|---|
| Thiamine·HCl | 0.2 mg/1 |
| Pyrodoxin·HCl | 0.25 mg/1 |
| Nicotinic acid | 1.3 mg/1 |
| Calcium pantothenate | 0.25 mg/1 |
| L-asparagine | 132.0 mg/1 |
| 2,4-D | 2.0 mg/1 |
| sucrose | 2% weight/volume |
| pH | 6.0 |

The friable embryogenic callus and medium are placed in a 100 ml Delong flask and are incubated at 27°C on a gyratory shaker at 130 rpm with indirect lighting from Agrolites for 16 hours a day for 7 days. Cultures were transferred every 7 days in a 2 to 5 culture to medium transfer into 50 ml of the same media in 250 ml Delong flasks.

Embryos are generated from the suspension cultures by removing a one ml aliquot from a 7 day old transfer and placing it on a solid plant growth medium such as MS medium or N6 medium containing no hormones or 0.01 mg/l 2,4-D and from 2 to 6% sucrose. The plates are maintained under the conditions described in Example 4 for approximately one week. Plants may be regenerated from the embryos by transferring the embryos to MS or N6 media containing no hormones and 2 to 6% sucrose as described in Example 6 below.

## EXAMPLE 4

### Formation of Embyros and Plantlets

A 15 day old suspension culture having a volume of approximately 25 ml was placed in a sterile centrifuge tube and the cells were spun down at 1000 rpm for 5 minutes. The supernatant was discarded and the cells were resuspended in 25 ml of hormone free N6 medium containing 6% sucrose. One ml of the resuspended cells was dispensed onto solid N6 medium containing 6% sucrose, no hormones, and 0.6% washed agar (K.C. Biologicals, Renex, Kansas). The plates were covered and maintained at 27°C under Agrolites at 1000 to 3000 lux for 16 hours a day. After 23 days, numerous globular structures grew on the hormone-free N6 medium containing 6% sucrose. These globular structures were removed and transferred to hormone-free MS medium with 2% sucrose and 0.1% charcoal. After 6 days four plantlets approximately 1 to 2 centimeters (cm) in length having shoots and roots were observed.

## EXAMPLE 5

### Regeneration of B73 Plantlets from Suspension Cultures

A B73 suspension (maintained as a suspension for approximately 4 months) was centrifuged at 1000 rpm for 5 minutes to pellet its cells. These cells were then weighed and diluted with hormone-free M.S. medium to a concentration of 100 mg suspended cells/ml hormone-free medium. One ml of medium (100 mg cells) was plated onto no hormone N6 medium with 2% sucrose, 0.1% charcoal, pH = 5.8.

These plates were incubated at 28°C in the dark. After one month, globular structures formed which were then transferred to fresh medium and placed under Agrolites. After approximately 3 weeks, many of these structures had developed into plantlets.

EXAMPLE 6

Regeneration of B73 Plants from Embryogenic Callus Culture

One hundred mg of friable B73 callus/petri plate was placed on N6 medium with 4% sucrose and no hormones. These plates were incubated at 28°C in the dark. After 27 days numerous plantlets were removed and transferred to plastic tissue culture boxes (about 3 inches x 3 inches x 4 inches) containing 50 ml of hormone free M.S. medium with 2% sucrose. After approximately one month, six of these plants were transferred to pots in the greenhouse. All of these plants were grown to maturity.

EXAMPLE 7

Isozyme Comparison of Embryogenic B73 Callus Culture
and Non-Embryogenic B73 tissue

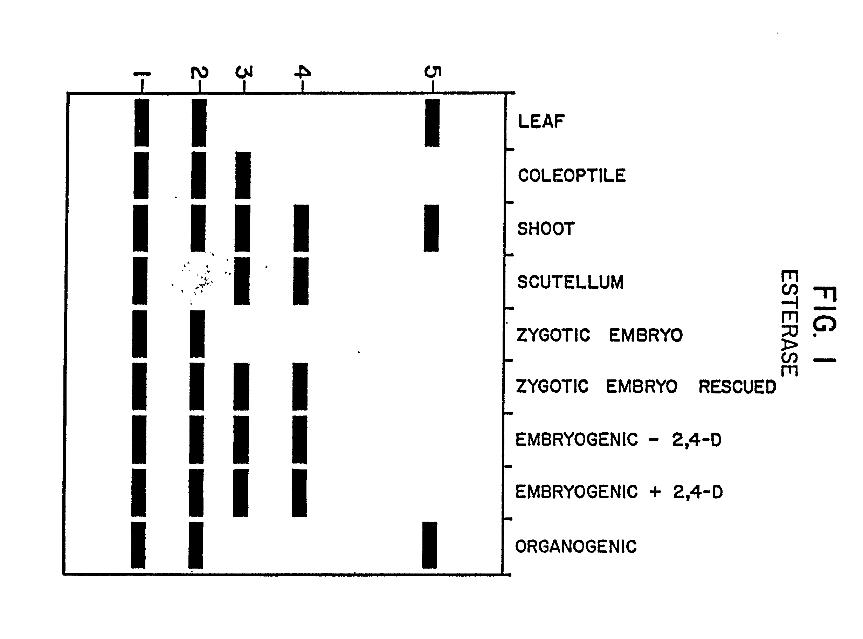

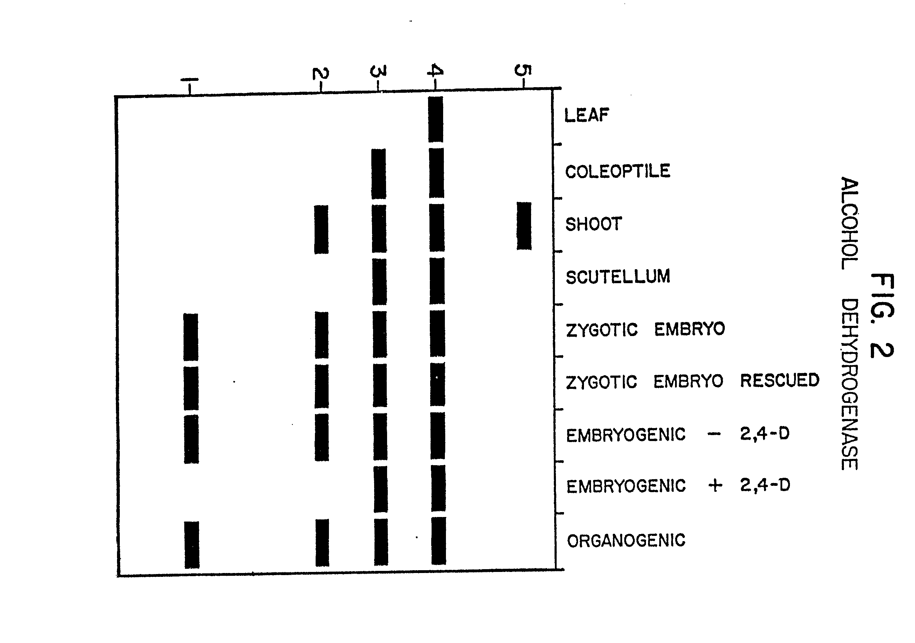

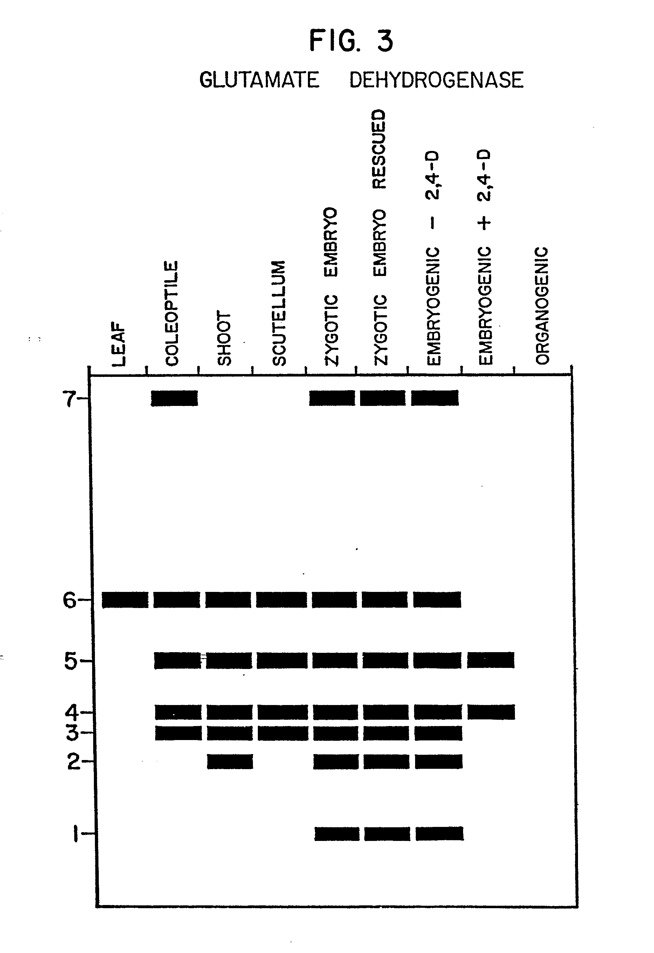

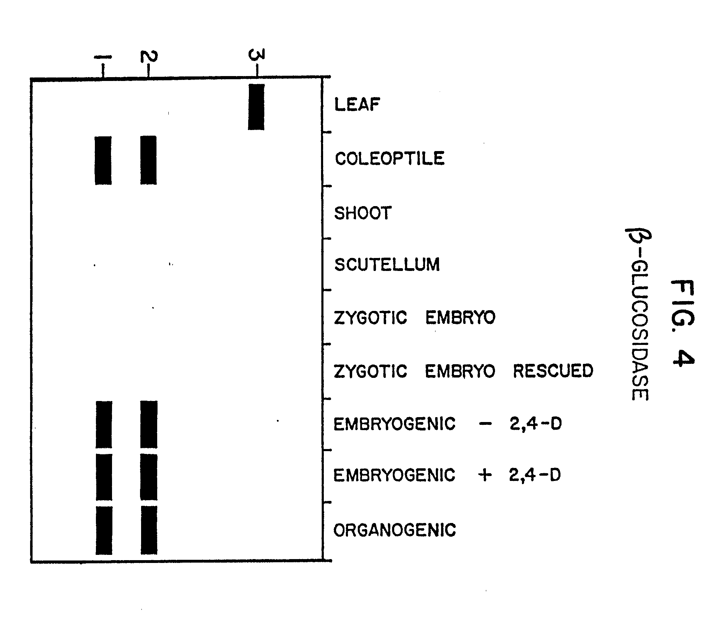

Between 0.5 and 5 grams of the corn inbred B73 tissue indicated below were placed in an equal weight of homogenization buffer containing 16.7% w/v sucrose, 8.3% w/v asorbic acid at pH 7.4, and was homogenized using a Porter tissue homogenizer. The homogenate was prepared for starch gel electrophoresis and the resulting gels were stained for esterase (Figure 1), alcohol dehydrogenase (Figure 2) glutamate dehydrogenase (Figure 3) and $\beta$-glucosidase (Figure 4) as described by Goodman et al. in Genetics, 96:697-70. In Figures 1-4 below, the tissues were grown as follows:

Leaves - taken directly from 6 week old greenhouse maintained B73 plants.

Coleoptile - taken from shoots six days after germination from seed.

Shoots - taken from mature B73 seeds one day after soaking in distilled water.

Scutellum - taken from mature B73 embryo one day after soaking in distilled water.

Zygotic Embryo - taken 19 days after pollination.

**0160390**

Immature Zygotic Embryo Rescued - taken 12 days after pollination and then
        transferred to the medium of Example 3 for 7 days.

Embryogenic - 2,4-D - friable embryogenic callus tissues of B73 that were
        maintained on the medium of Example 3 without 2,4-D for 19 days.

Embryogenic + 2,4-D - friable embryogenic tissues of B73 that were maintained on the medium of Example 3.

Organogenic - organogenic tissue of B73 that were maintained on the medium
        of Example 3.


        Figures 1-4 are representations of starch gels run for the indicated enzymes.  The gels patterns show the following results:


        Esterase - Figure 1 - Embryogenic cultures gave the same isozyme
patterns as rescued zygotic embroyos and organogenic cultures gave the
same isozyme pattern as mature leaves.


        Alcohol dehydrogenase - Figure 2 - organogenic cultures and
embryogenic cultures on medium without 2,4-D gave the same isozyme pattern
as zygotic embryos, but embryogenic cultures on the same medium containing
2,4-D did not.


        Glutamate dehydrogenase - Figure 3 - embryogenic cultures on
medium without 2,4-D gave the same pattern as zygotic embryos, but embryogenic cultures on maintenance medium with 2,4-D did not.


        $\beta$-Glucosidase - Figure 4 - embryogenic and organogenic culture
gave the same pattern as coleoptiles but a different pattern than zygotic
embryos.


        These results support the characterization that regeneration
from embryogenic cultures proceeds by a type of somatic embryogenesis.
The presence of isozymes usually associated with coleoptiles suggests that
the timing of embryogenic developmental events may be different than that
of  of zygotic embryos.

## EXAMPLE 8

### Characterization of Polysaccharide Secretion Product of Embryogenic B73 Tissues

Ten ml of a 7-day-old embryogenic cell suspension of corn inbred B73 was transferred into a fresh 50 ml aliquot of the medium of Example 3. The cultures were grown in the light at 27°C in a shaker at 130 rpm. After 7 days, cells and debris were removed from the medium by centrifugation or filtration. The remaining clear culture medium was then diluted with 3 volumes of ethanol (95% v/v) at room temperature with stirring. A white fibrous precipitate formed, was collected on a glass rod, washed in absolute ethanol and dried in a vacuum dessicator oven. The dried precipitate was hydrolyzed in 1 normal hydrochloric acid for 6 hours at 100°C and the component sugars were analyzed by $^{13}C$ natural abundance nuclear magnetic resonance ($^{13}C$-NMR) on a Varian XL-200 NMR spectrometer.

The polysaccharide was identified as a polymer comprising galactose and mannose in about a 4:1 glactose:mannose ratio. The polymer has a composition different from maize root slime, which contains a glucose-rich polymer abundant in fucose and galacturonic acid, and corn hull-gum, which contains xylose, arabinose and galatose in a 7:5:1 ratio (see Smith, F. et al., The Chemistry of Plant Gums and Mucilages and Some Related Polysaccharides, Reinhold Publishing Corp., New York).

## EXAMPLE 9

### Carbon Source Utilization

Embryogenic suspension cultures of corn inbred B73 were washed in carbohydrate-free medium and plated onto the medium of Example 3 that had been solidified with 0.6% washed agar and contained 1% w/v of the filter-sterilized test compounds listed in Table I below. Colony growth was assessed visually after 2 weeks and 9 weeks growth in the dark at 27°C.

The toxicity of galactose in the presence of sucrose was tested on embryogenic suspension cultures of corn inbred B73 by incorporating galactose (0.1 to 6% w/v) into the medium of Example 3 containing 2% w/v sucrose. Growth was measured by packed cell volume (PCV) after 7 days incubation on a shaker at 27°C in the light.

## TABLE I

Growth of Embryogenic Cells of Corn Inbred B73 on Various Carbon Sources
(1% w/v)

| Sole Carbon Source | B73 Cell Growth Relative to Carbohydrate-Free Control |
|---|---|
| | 2 Weeks |
| sucrose | +++ |
| D-glucose | +++ |
| soluble starch | +++ |
| D-(+)-galactose | + |
| -lactose | 0 |
| D-sorbitol | + |
| L-proline | 0 |
| 0 (control) | 0 |
| L-(+)-arabinose | 0 |
| myo-inositol | 0 |
| -D-(+)-fucose | 0 |
| L-glycine | 0 |
| L-arginine | 0 |
| L-(-)-xylose | 0 |
| D-(+)-mannose | 0 |
| DL-malic acid | 0 |
| D-mannitol | 0 |
| L-(-)-sorbose | 0 |
| -L-rhamnose | 0 |
| L-glutamic acid | 0 |

+ = Visibly detectable growth.

Most of the carbon sources did not support growth of the cells
and the best growth was obtained with sucrose and glucose.  Soluble starch
supported good growth of the cell line after an initial lag period.  The
cell line showed limited growth on sorbitol but no growth on proline and
lactose.  The embryogenic corn cell line of B73 showed limited growth
response to galactose.

The embryogenic callus of corn inbred B73 which grew on starch-containing plates appeared to lack the galactomannan secretion present in the plates containing sucrose or glucose. Suspension cultures of the same tissue growing on starch produced no detectable amounts of galactomannan.

## EXAMPLE 10

Plants obtained in Example 6 were grown in the greenhouse and were either self-pollinated, backcrossed to the parent B73 line, or sibling-pollinated with sister regenerated plants (designated $R_1S_1$ for self-pollinated, $R_1BC_1$ for backcrossed and $R_1Sib_1$ for sibling pollinated). The corn progenies were produced from four regenerated plants. Two of the regenerated plants were self-pollinated using the main stem ear and a tiller tassel. Because of asynchronous emergence of tassels and silks and frequent occurence of tassel-ears, the remaining regenerated corn plants could often not be self-pollinated, but instead were used as female parents in crosses to seed-grown B73.

The seeds produced were grown out in the field between December, 1983 and March 1984. Plants were evaluated in early February, which was approximately two weeks post-pollination for the corn. A total of ten rows of regenerant progeny were planted out ear-to-row. In addition, two seed-grown plants used as male parents in crosses with the regenerants were also self-pollinated and used as check rows. Traits examined included: plant height, ear height, stem diameter, leaf number and any evidence of single gene mutations affecting plant appearance. While some rows showed evidence of salt stress, no differences could be seen between the $R_1S_1$, $R_1BC_1$, and $R_1Sib_1$ progeny and the seed-grown check rows.

From the foregoing it will be apparent to those skilled in the art that embryogenic tissues and cell suspensions of corn inbred B73 will enable plant breeders, practitioners of methods of plant tissue and cell culture, and plant molecular biologists to carry out major improvements in this valuable and widely used corn inbred. The embryogenic cultures of corn inbred B73 provided by the instant invention make it possible, for example, to subject these cultures to in vitro mutagenesis to induce variation in corn plants regenerated therefrom. Methods for chemical and physical mutagenesis as well as cell culture techniques for the recovery of desirable mutants are well known to those skilled in the art (see for example: Popova, J., "Possibilities of Inducing Economically Useful Mutations in Maize Seed by Treatment with Ethyl Methanesulphonate," Genetic Plant Breeding, 8:282-290 (1975); Balint et al., "Examination of WF9 Mutants Sublines for Lodging and Fusarim Resistance," Induced Mutations Against Plant Diseases, pp. 489-494 IAEA, Vienna; Weber and Lark, "An Efficient Plating System for Rapid Isolation of Mutants from Plant Cell Suspensions," Theor. Appl. Genetics, 55:81-86 (1979); and Thomas, J.A. et al., "Improvement of Crop Plants via Single Cells in Vitro - An Assessment," Z. Planzenzücht, 82:1-3 (1979). Thus, the invention is considered to encompass mutagenized B73 tissue cultures and cell suspensions and corn plants regenerated therefrom and the mutant or variant progeny of the regenerated corn plants.

The ability to regenerate whole plants from the embryogenic tissue and cell suspension cultures of corn inbred B73 provided by the instant invention also enables those skilled in the art to carry out in vitro selections for desirable traits or against undesirable traits. (See Hibbert et al. and Bengenbach et al., both mentioned hereinabove.) Thus, using the embryogenic tissue or cell suspensions cultures of corn inbred B73 of the instant invention, it is now possible to expose cultures of · corn inbred B73 to selective agents for example herbicides and pathotoxins, to select tissues and cells resistant to the selective agents and to regenerate plants resistant to such selective agents. Thus, considered within the scope of the instant invention are embryogenic tissues and cells of corn inbred B73 selected in vitro, the corn plants and seed · thereof regenerated therefrom and the progeny of the regenerated corn plants.

It will also be readily apparent to those skilled in the art that embryogenic tissue and cell suspension culture of corn inbred B73 provides the opportunity for the generation of somaclonal variants in corn inbred B73 by regenerating plants from embryogenic tissue culture and cell suspension cultures of corn inbred B73 provided by the instant invention. Such stable somaclonal variants are well known to those skilled in the art as exemplified in Edallo et al., "Chromosomal Variation and Frequency of Spontaneous Mutation Associated with the in vitro Culture and Plant Regeneration in Maize," _Maydica_ _26_, pp. 39-56 (1981); Beckert et al., "Etude de la Variabilite Genetique Obtenue Chez le Mais Apres Callogenese et Regeneration de Plants _in vitro_, Agronomie _3_, pp. 9-18 (1983); and Larkin et al., "Somaclonal Variation - A Novel Source of Variability from Cell Cultures for Plant Improvement," _Theor. Appl. Genet._ 60, pp. 197214 (1981). A large body of literature in fact suggests that plants regenerated from culture are characterized by an unexpectedly high rate of stable propagatable phenotypic variation. See Rice, "Tissue Culture Inbred Genetic Variation in Regenerated Maize Inbred," 37th Ann. Corn & Sorghum Research Conference (1983). Thus it is now possible to provide progeny with genetic variations arising from somaclonal variants regenerated from corn inbred B73. Such characteristics may be stably maintained in the second generation progeny of the regenerated plants and will generally exhibit either Mendelian segregation (e.g., 3:1 ratio for single gene traits) or uniform expression since somaclonal variation arises in diploid cells and may generate homozygous variants. Thus, the invention is considered to encompass somaclonal variants of the embryogenic callus and cell suspension of corn inbred B73, corn plants and the seed thereof regenerated therefrom and the progeny of the regenerated corn plants.

CLAIMS:

1. Embryogenic callus and embryogenic cell suspensions of corn inbred B73 and the clones thereof.

2. Corn plants and the seed thereof regenerated from embryogenic callus and embryogenic cell suspensions of corn inbred B73 and the clones thereof.

3. Mutagenized embryogenic callus and mutagenized cell suspensions of corn inbred B73 and clones thereof.

4. Corn plants and the seed thereof regenerated from mutagenized embryogenic callus and mutagenized cell suspensions of corn inbred B73 and the clones thereof.

5. Progeny of corn plants regenerated from embryogenic callus and embryogenic cell suspensions of corn inbred B73 and clones thereof, said progeny including mutants and variant progeny.

6. Embryogenic callus of corn inbred B73 having the characteristics of an embryogenic callus of corn inbred B73 deposited in the American Type Culture Collection under ATCC Accession Number 40116, and cell suspensions derived from said embryogenic callus and clones of said embryogenic callus or embryogenic cell suspensions.

7. Corn plants and the seed thereof regenerated from the embyrogenic callus and embryogenic cell suspensions and clones of said embryogenic callus or embryogenic cell suspensions of Claim 4.

8. Mutagenized embryogenic callus of corn inbred B73 said embryogenic callus of corn inbred B73 having the characteristics of an embryogenic callus of corn inbred B73 deposited in the American Type Culture Collection under ATCC Accession Number 40116, mutagenized cell suspensions derived from said embryogenic callus and clones of said mutagenized embryogenic callus or embryogenic cell suspensions.

9. Corn plants and the seed thereof regenerated from the mutagenized embryogenic callus and mutagenized cell suspensions and clones of said mutagenized embryogenic callus and mutagenized embryogenic cell suspensions of Claim 8.

10. Progeny of corn plants regenerated from embryogenic callus having the characteristics of an embryogenic callus of corn inbred B73 deposited in the American Type Culture Collection under ATCC Accession Number 40116 or regenerated from cell suspensions derived from said embryogenic callus having the characteristics of an embryogenic callus of corn inbred B73 deposited in the American Type Culture Collection under ATCC Accession Number and clones thereof, said progeny including mutants and variant progeny.

11. An embryogenic callus of corn inbred B73 as deposited in the American Type Culture Collection under ATCC Accession Number 40116, cell suspensions derived from said embryogenic callus and clones thereof.

12. Corn plants and seed thereof regenerated from the embryogenic callus of corn inbred B73 as deposited in the American Type Culture Collection under ATCC Accession Number 40116 cell suspensions derived from said embyrogenic callus and clones thereof.

13. Mutagenized embryogenic callus of corn inbred B73 as deposited in the American Type Culture Collection under ATCC Accession Number 40116, mutagenized cell suspensions derived from said embryogenic callus and clones thereof.

14. Corn plants and the seed thereof regenerated from the mutagenized callus, mutagenized cell suspensions and clones of said mutagenized embryogenic callus and mutagenized embryogenic cell suspensions of Claim 13.

15. Progeny of corn plants regenerated from the embryogenic callus corn inbred B73 as deposited in the American Type Culture Collection under ATCC Accession Number 40116, cell suspensions derived from said embryogenic callus and clones thereof, said progeny including mutant and variant progency.

FIG. I

ESTERASE

# FIG. 2

## ALCOHOL DEHYDROGENASE

# FIG. 3

## GLUTAMATE DEHYDROGENASE

0160390

# FIG. 4

β-GLUCOSIDASE